Europäisches Patentamt

European Patent Office

Office européen des brevets

(11) Publication number: **0 534 526 A1**

## EUROPEAN PATENT APPLICATION

(21) Application number: **92202796.6**

(22) Date of filing: **14.09.92**

(51) Int. Cl.5: **C12N 15/31**, A61K 39/02, C07K 13/00

(30) Priority: **25.09.91 EP 91202478**
**24.07.92 EP 92202273**

(43) Date of publication of application:
**31.03.93 Bulletin 93/13**

(84) Designated Contracting States:
**AT BE CH DE DK ES FR GB GR IE IT LI LU NL PT SE**

(71) Applicant: **DUPHAR INTERNATIONAL RESEARCH B.V**
**C.J. van Houtenlaan 36**
**NL-1380 AC Weesp(NL)**

(72) Inventor: **Koopman, Marcel B.H., c/o**
**Octrooibureau Zoan b.v.**
**P.O. Box 140**
**NL-1380 AC Weesp(NL)**
Inventor: **Kusters, Johannes G., c/o**
**Octrooibureau Zoan b.v.**
**P.O. Box 140**
**NL-1380 AC Weesp(NL)**

(74) Representative: **Breepoel, Peter M.**
**Octrooibureau Zoan B.V. P.O. Box 140**
**NL-1380 AC Weesp (NL)**

(54) **Treponema hyodysenteriae vaccine.**

(57) The present invention is concerned with vaccine for combating Treponema hyodysenteriae infection in swine containing proteins or polypeptides typical of the endoflagellum sheath protein of Treponema hyodysenteriae or containing recombinant polynucleotides having as part thereof a polynucleotide coding for said protein or polypeptide, and also is concerned with the preparation of said proteins, polypeptides and polynucleotides.

EP 0 534 526 A1

The present invention is concerned with a vaccine for combating Treponema hyodysenteriae infection and with recombinant polynucleotides and polypeptides for the preparation of such a vaccine.

Treponema hyodysenteriae, the etiological agent of swine dysentery is an anaerobic, beta-hemolytic spirochete found in the porcine large intestine. It is reported that by a cork-screw like movement the Treponema hyodysenteriae invades the colon of pigs. Upon infection this pathogen secretes a hemolysin which is thought to play an essential role in the pathogenesis of the disease. The disease is characterized by a mucohemorrhagic diarrhoea. This seems to be associated with the extensive superficial necrosis of the luminal epithelial lining and of the crypts of Lieberkuhn.

Treponema hyodysenteriae produces endoflagella which, next to hemolysin, are considered to act as virulence factors. An endoflagellum is composed of several endoflagellum proteins. It is suggested that the characteristic cork-screw like movement of Treponema hyodysenteriae, which helps the organism to successfully settle in the colon of pigs, is made possible by bundles of seven to nine endoflagella which are situated underneath the outer membrane of the bacterium. Inhibition of the function of the endoflagella are considered to prevent the successful establishment of Treponema hyodysenteriae and hence protect the pig against swine dysentery.

It has been found that the endoflagellum consists of at least four proteins: a 43 kD protein forms the sheath of the flagellum, whereas three proteins (37, 34 and 32 kD) make up its core. Antibodies raised against each of the flagellar proteins showed to be bactericidal for Treponema hyodysenteriae in vitro. The gene coding for the 43 kD sheath protein was cloned, and the nucleotide sequence and deduced amino acid sequence were established. These are represented in SEQUENCE ID NO: 1.

Accordingly, the present invention is concerned with a substantially pure preparation of Treponema hyodysenteriae endoflagellum sheath protein or polypeptide endoflagellum sheath protein fragments (endoflagellum sheath polypeptides) having at least part of the amino acid sequence of SEQUENCE ID NO: 1.

More in particular, the present invention is based on the finding that the endoflagellum sheath protein and polypeptides of interest are able to elicit an immune response against Treponema hyodysenteriae upon administration to swines.

Furthermore, the present invention is not only concerned with the endoflagellum sheath protein and polypeptides but also with DNA of SEQUENCE ID NO: 1 and fragments thereof, and with polynucleotides which hybridize to said DNA and fragments thereof and which code for a polypeptide having the immunogenic properties of the endoflagellum sheath protein of Treponema hyodysenteriae.

The present invention is concerned also with a polynucleotide which codes for a polypeptide having the immunogenic properties of the endoflagellum sheath protein of Treponema hyodysenteriae wherein at least part of the codons of the DNA of SEQUENCE ID NO: 1 or of the fragments thereof, or of the abovementioned hybridizing polynucleotide is replaced by alternative codons for the same amino acid.

Small antigens often are useless as immunogens. Therefore the endoflagellum sheath protein or polypeptides may be prepared as homopolymers (a multitude of indentical endo-flagellum sheath polypeptides coupled) or heteropolymers (one or more endoflagellum sheath polypeptide coupled to one or more different endoflagellum sheath polypeptides, or coupled to one or more different polypeptides characteristic of Treponema hyodysenteriae or an other pathogen), or may be coupled to one or more other compounds in order to enhance immunogenicity.

According to the present invention the endoflagellum sheath polypeptide, in any of the modifications mentioned above, may be prepared by recombinant DNA techniques known in the art or may be prepared synthetically, e.g. by homogeneous or by solid state polypeptide synthesis known in the art.

The particular amino acid sequences of suitable endoflagellum sheath polypeptides may be derived from the amino acid sequence according to SEQUENCE ID NO: 1 and or from the spatial configuration of the endoflagellum sheath protein. Suitable endoflagellum sheath polypeptides may be selected from the most hydrophilic parts of the endoflagellum sheath protein, e.g. by applying the technique described by Hopp and Woods (T.P. Hopp and K.R. Woods (1981): Proc. Natl. Acad. Sci., USA. 78, 3824-3828). Another suitable method for selecting such polypeptides is described by Chou and Fasman (P.Y. Chou and G.D. Fasman (1987): Advances in Enzymology 47, 45-148).

A number of methods has been developed to predict the location of immunogenically important epitopes on proteins. The outcome of the combined predictions gives a good forecast of antigenic sites.

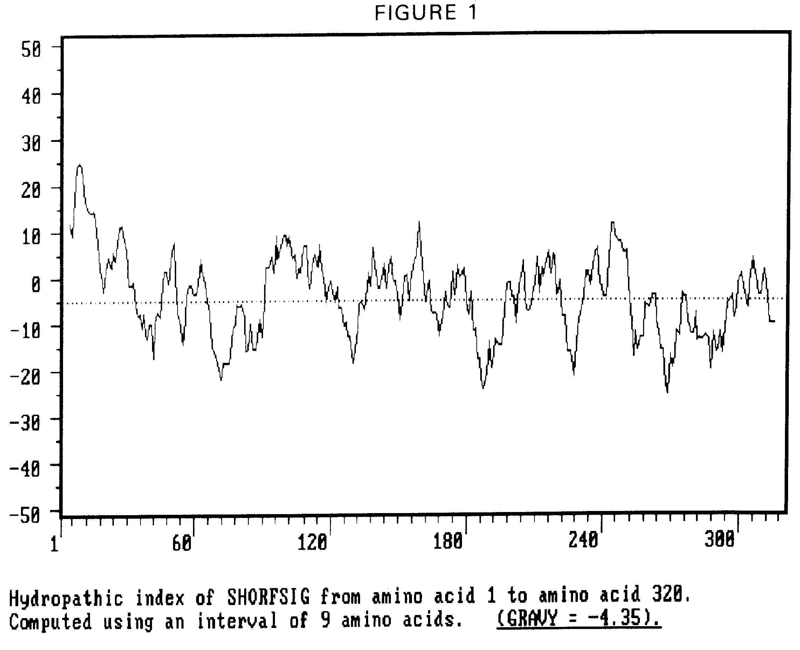

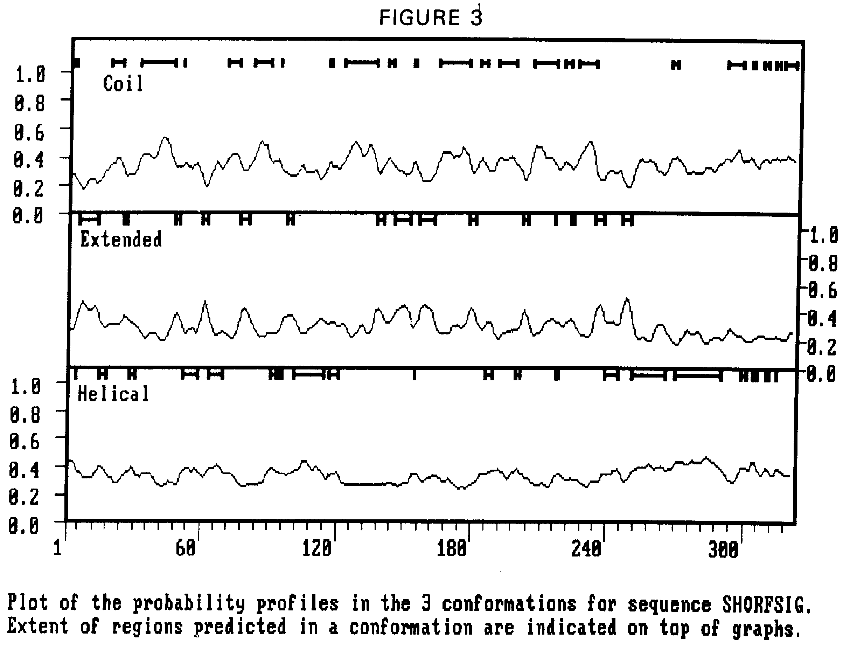

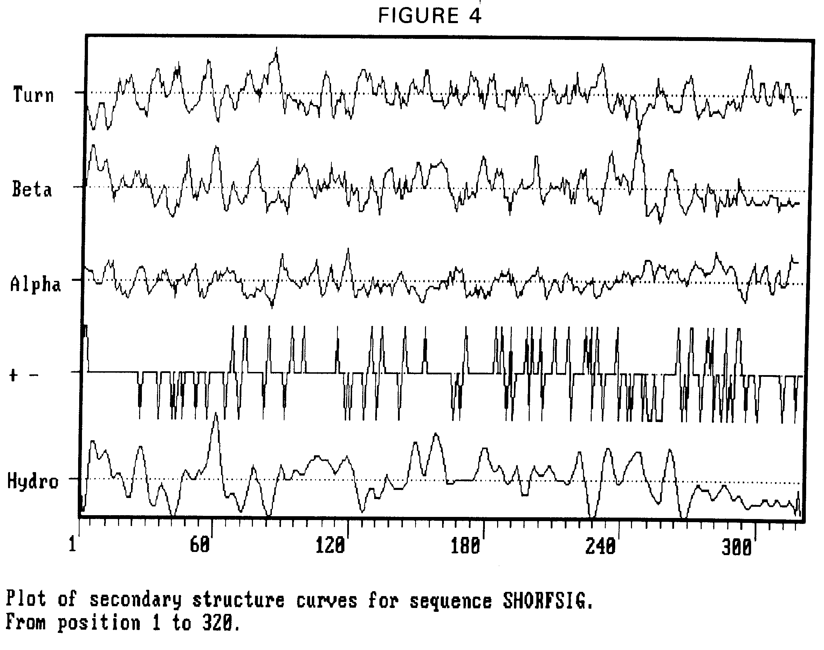

Figures 1 - 4 show the various algorithms that have been used to finally predict the antigenically important regions at the Treponema hyodysenteriae sheath protein.

Figure 1 represents the hydropathic index of the protein sequence of Treponema hyodysenteriae sheath protein from amino acid 1 to amino acid 320, computed using an interval of 9 amino acids.

Figure 2 represents a hydrophylicity profile of the protein sequence of Treponema hyodysenteriae sheath protein, computed using an average group length of 6 amino acids. This results in a prediction of antigenic determinants according to Hopp and Woods, 1981.

Figure 3 represents a plot of the probablity profiles in the 3 conformations for the sequence of Treponema hyodysenteriae sheath protein, according to O. Gascuel and J.L. Golmard, 1988 (CABIOS 4, 357-365). The extent of the regions predicted in each of the conformations are indicated on top of the graphs.

Figure 4 presents a prediction of the secondary structure of the sequence of Treponema hyodysenteriae sheath protein according to J. Novotny and C. Auffray, 1984 (Nucleic Acids Research 12, 243-255).

Additional information on the location of relevant epitopes can be obtained using the PEPSCAN-method, developed by Geysen and Meloen (H.M. Geysen, R.H. Meloen and S.J. Barteling, S.J. (1984): Proc. Natl. Acad. Sci., 81(13); 3998-4002). This method uses synthetic peptides of sufficient length to react in an enzyme-linked immunosorbent assay. These peptides are synthesised according to a given DNA-sequence. They are characterised by the fact that the first peptide covers amino acid nr. 1-9, the second peptide covers amino acid nr. 2-10 etc.

Each peptide is tested for its reactivity with antisera or monoclonal antibodies. Reactive peptides must then represent an immunogenic epitope.

To identify immunoreactive epitopes (and to correlate these reactivities with the physical map of the sheath protein) DNA fragments from the *sheath protein* gene will be expressed in the pEX plasmids (K. Stanley and J.P. Luzio, 1984. EMBO J. 3, 1429-1434, and J.G. Kusters, E.J. Jager and B.A.M. Van der Zeijst, 1989. Nucl. Acids Res., 17, 8007). In this system, heterologous expression leads to the synthesis of a C-terminal extension of the cro-$\beta$-galactosidase hybrid protein. Restriction-endonuclease sites in the sheath protein DNA sequences can be used to obtain fragments of the sheath protein gene for insertion into the pEX plasmids. pEX clones synthesizing fusion proteins derived from different overlapping regions of the sheath protein will then be used for further characterization. The pEX encoded sheath protein fragments are purified, fractionated by polyacrylamide gel electrophoresis, and blotted to nitrocellulose membranes. These membranes are then reacted with sera from immune pigs. Only the fragments containing the immuno reactive epitopes will react with these sera. To delineate the minimal lenght of the epitopes, the DNA inserts of the reactive clones are progressively shortened by Exonuclease III digestion, or by cloning synthetic oligonucleotides encoding small overlaping parts of the sheath protein (J.G. Kusters, E.J. Jager, G. Koch, J.A. Lenstra, G. Koch, W.P.A. Posthumus, R.H. Meloen and B.A.M. Van der Zeijst, 1989. J. Immunol., 143, 2692-2698). The epitopes are then tested for their protective effects.

According to a particular embodiment of the present invention an endoflagellum sheath-protein-specific polypeptide is produced by expression of a polynucleotide having at least part of the polynucleotide SEQUENCE ID NO: 1 forming part of a recombinant polynucleotide. The recombinant polynucleotide preferably may be based on a vector with a Treponema hyodysenteriae-specific polynucleotide fragment inserted therein. Suitable vectors are plasmids, bacteriophages, cosmids, viruses, minichromosomes or stably integrating vectors; the latter in particular for plant or animal cells. Generally these vectors need to have the property of autonomous replication except for the stably integrating vectors which insert themselves in the genetic material of the host cell and replicate with the host's genetic material. Suitable host cells may be either prokaryotic or eukaryotic, such as bacteria, yeasts, mycoplasms, algae, plant cells or animal cells; the plant cells or animal cells may be cultivated in vitro or may form part of an intact plant or animal, respectively. The recombinant polynucleotide may contain as an insert a complete polynucleotide coding for endoflagellum sheath protein or a fragment thereof. The insert may comprise a single coding sequence, or multiple copies of the same coding sequence, or a hybrid polynucleotide containing at least one endoflagellum sheath protein coding sequence and at least one second sequence such as a different part of the endoflagellum sheath protein coding sequence or a polynucleotide coding for a protein characteristic of an other pathogen or for an inert protein functioning as a carrier for a small endoflagellum sheath-polypeptide.

A specific case of the above embodiment is concerned with recombinant polynucleotides with viral vectors, directly useful as so-called vector vaccines. The viruses applicable for this purpose should have the ability to replicate in the animals to be immunized, i.e. in swines. These viruses, furthermore, should possess a genomic region suitable for insertion of a foreign gene (e.g. coding for the Treponema hyodysenteriae-endoflagellum sheath protein or polypeptide) which is also to be expressed in the vaccinated animal. Suitable viruses for this purpose are for example enteral viruses such as certain adeno viruses.

As was indicated above, the proteins and polypeptides, and the recombinant polynucleotides according to the invention are useful in the preparation of vaccines. Hence, these vaccines also form part of the invention.

A particular application of the present invention is concerned with bacterial vector vaccines. Herein bacteria capable of colonizing swines are transformed in order to enable them to express an endoflagellum sheath protein or endoflagellum sheath polypeptide in such a way that it will lead to an immunogenic respons against Treponema hyodysenteriae. Suitable bacteria for this purpose are e.g. Salmonella bacteria.

A vaccine according to the invention may also contain auxiliary vaccine constituents, such as carriers buffers, stabilizers, solubilizers, adjuvants and preservatives. Advantageously these vaccines are freeze-dried products which are reconstituted by the addition of a suitable liquid (water, buffer) prior to application.

The vaccine can be applied e.g. orally, intranasally or intramuscularly.

The vaccine may additionally contain other immunogens for swines, such as immunogenic material characteristic of viruses such as pseudorabies virus, influenza virus, transmissible gastroenteritis virus, parvo virus, porcine endemic diarhoea virus, hog cholera virus, or immunogenic material characteristic of mycoplasms, such as Mycoplasma hyopneumoniae and mycoplasma lyorhinis, or immunogenic material characteristic of bacteria, such as Escherichia coli, Bordetella bronchiseptica, Leptospira, Actinobaccilus pleuropneumoniae, Pasteurella multocida, Streptococcus suis.

The invention is illustrated by the following working Examples.

EXAMPLE 1

BIOCHEMICAL AND STRUCTURAL ANALYSIS OF PERIPLASMIC FLAGELLA FROM TREPONEMA HYODYSENTERIAE

Materials and methods

Bacterial strains and growth conditions

*Treponema hyodysenteriae* was cultured at 40°C in an anaerobic hood, either in 250 ml trypticase soy broth suplemented with 10% fetal calf serum, 0,05% RNA core type II C from Torula yeast (Sigma) and 400 ug/ml spectinomycin, or on trypticase soy agar (Becton Dickinson, Cockeysville, USA) plates supplemented with 10% sheep blood, 400 ug/ml spectinomycine and 0.06% yeast (Oxoid, Basingstoke, England). Most experiments presented here were performed with *Treponema hyodysenteriae* strain C5, which was originally isolated from the colon of a pig severely affected by swine dysentery. It shows a strong hemolysis on blood agar plates and is a representative of the most prevailing type of *Treponema hyodysenteriae* in the Netherlands, as judged by restriction enzyme analysis of its genome.

Flagella isolation and purification

Periplasmic flagella were prepared by a modification of the method described by Kent (K.A. Kent, R. Sellwood, R.M. Lemcke, M.R. Burrows and R.J. Lysons. 1989. Analysis of the axial filaments of *Treponema hyodysenteriae* by SDS-PAGE and immunoblotting. J. Gen. Microbiol. 135:1625-1632). Bacterial cultures were harvested by centrifugation at 15,000 g for 30 min at 4°C, washed once with phosphate-buffered saline (PBS) and pellets were suspended in the same buffer. Outer envelopes were removed by the addition of sodium dodecyl sulphate (SDS) to 0.1 % and incubating the suspension for 30 min at room temperature with gentle shaking. The organisms were collected by centrifugation at 25,000 g for 30 minutes at 4°C and resuspended in PBS. Flagella were removed from the cells by shearing in a blender and separated from the bacteria by centrifugation at 30,000 g for 30 min at 4°C. Sodium lauroyl sarkosinate (Sarkosyl) was added to the supernatant (0,2% (w/v)) and filaments were sedimented by centrifugation at 94,000 g for 60 min at 4°C and resuspended in water. Flagellar proteins were stored at -20°C.

Electrophoresis and Western blotting

Proteins were separated on 12.5% SDS-polyacylamide gel electrophoresis (SDS-PAGE) essentially as established by Laemmli (U.K. Laemmli, 1970. Cleavage of structural proteins during the assembly of the head of bacteriophage T4. Nature (London) 277:680-685.). Proteins were visualized by Coomassie Blue R 250 staining and molecular weights were estimated through comparison with low molecular mass markers (Pharmacia). Proteins were transferred to nitrocellulose sheets (Schleicher & Schuell, Dassel, Germany) according to the method of Towbin et al. (H. Towbin, T. Staehelin, and J. Gordon. 1979. Electrophoretic transfer of proteins from polyacrylamide gels to nitrocellulose sheets: procedure and some applications.

Proc. Natl. Acad. Sci. USA 76:4350-4354) using a Bio-Rad Transblot cell (Bio-Rad, Hertfordshire, England). After transfer, the nitrocellulose was blocked with 0,5% gelatine and 0,5% Tween-20 in PBS (PBS$^+$) for 1 hour on a rocking platform. The nitrocellulose was then incubated with an appropriate dilution of antisera in PBS$^+$ for 1 hour. The filter was washed three times for 5 min with PBS$^+$ and further incubated for 1 hour with a 1:300 dilution of an alkaline phospatase conjugate of either goat anti-rabbit immunoglobuling G (Sigma) or a 1:2000 dilution of goat anti-mouse immunoglobulin G (Promega) in PBS$^+$. After three washings with PBS$^+$ bound antibodies were visualized by development in nitro blue tetrazolium (0,37 mmol/l) and 5-bromo-4-chloro-3-indolyl phosphate (0,34 mmol/l) solubilized in 100 mmol/l Tris/HCl, pH 9,5, 100 mmol/l NaCl, 5 mmol/l MgCl2. Incubations and washings were performed at room temperature on a rocking platform.

Electron microscopy

*Treponema hyodysenteriae* cells were grown for three days on trypticase soy agar plates and collected in two ml 0.03% sucrose, 0.01 mol/l MgCl2, 0.01 mol/l CaCl2. Samples were applied to pioloform coated copper grids (200 mesh, Biorad) for 10 minutes, then washed briefly 3 times on drops of destilled water and negatively stained with phosphotungsten acid (PTA). For immunogold labeling bacteria were adsorbed onto grids and rinsed briefly 3 times with destilled water. Subsequently, they were incubated for 30 minutes on drops of 1:1000 diluted antiserum in PBT (PBS containing 1% BSA, 0,05% Tween and 0.1 % gelatin). After 3 rinses with PBT, grids were further incubated for 30 minutes with protein A conjugated with 10 nm gold particles (Janssen Life Sciences Products, Beerse, Belgium). After rinsing the grids twice with destilled water the samples were negatively stained with PTA as described. All samples were examined with a Philips 201 electron microscope operating at 60 kV and an objective aperture of 30 $\mu$m.

Antisera

Rabbit antisera to the individual components of purified flagella from *Treponema hyodysenteriae* C5 were obtained as follows. Approximately 600 ug of flagellar proteins was separated by SDS-PAGE, blotted onto nitrocellulose filters and visualized by staining with 0,6% Ponceau S in 3% TCA. The area of the filters containing the desired protein was cut out, shock-frozen in liquid nitrogen and homogenized with a Braun mikro dismembrator. The homogenized material was suspended in 3 ml destilled water and stored at -20°C until further use. For immunization, 1,5 ml of this material was mixed with an equal volume of Freunds complete adjuvant and inoculated intramuscularly in a New Zealand White rabbit on day 0. The rabbit was boostered with 1,5 ml of homogenized antigen/nitrocellulose in an equal volume of Freunds incomplete adjuvant on day 28. Sera were collected at days -1, 20, 34, 41 and day 48, when rabbits were exsanguinated.

Rabbit antiserum (R$\alpha$TPRPF) to *Treponema phagedenis* biotype Reiter periplasmic flagella was a gift from G. Noordhoek, RIVM Bilthoven, the Netherlands and rabbit serum (R$\alpha$SAPF) and mouse monoclonals .1H11G7 and .2A6B4 against *Spirochaeta aurantia* flagella were obtained from P. Greenberg, University of Iowa, United States (B. Brahamsha and E.P. Greenberg. 1988. A biochemical and cytological analysis of the complex periplasmic flagella from *Spirochaeta aurantia*. J. Bact. 170;4023-4032). Mouse monoclonal MCC9 to the 37 kb *Treponema pallidum* flagellar protein was from C. W. Penn, University of Birmingham, United Kingdom (M.J. Bailey, A. Cockayne, and C.W. Penn. 1987. Production of murine monoclonal antibodies to the major axial filament polypeptide of *Treponema pallidum*. J. Gen. Microbiol. 133:1-805-1813).

N-terminal sequence analysis

Periplasmic flagellar proteins were separated by SDS-PAGE and electroblotted on polyvinylidene difluoride membranes (Immobilon Transfer Membranes; Millipore, Bedford, Mass.) in 10 mmol/l 3-cyclohexylamino-1,1-propanesulfonic acid, pH 11.0 and 10% methanol. The blotted proteins were visualized by Coomassie brilliant blue R250 staining, bands containing the proteins of interest cut out and the amino acid sequence of the blotted proteins determined with an Applied Biosystems Model 470A Protein Sequencer, on-line equipped with a model 120A PTH Analyzer.

RESULTS

Purification and SDS-PAGE analysis

In order to identify the components that constitute the periplasmic flagella from *Treponema hyodysenteriae* the flagellar proteins from *Treponema hyodysenteriae* strain CS were purified. SDS-PAGE analysis of the flagellar preparation revealed the presence of five major polypeptides with an apparent molecular weight of 44, 37, 35, 34 and 32 kD, suggesting that these are the major components of the flagella. In some preparations two additional bands at 30 and 28 kD were visible. Since these bands were of low intensity they probably are not major constituents of the flagella and may represent contaminants.

Immunoblot analysis

To confirm the flagellar nature of the five major polypeptides in the flagellar preparation we tested their reactivity on immunoblots with sera and monoclonal antibodies against flagellar proteins of related spirochetes. With the exception of the 30 and 28 kD proteins, all proteins present in the flagellar preparation reacted with one or more of these sera (Table 1). Reactivity was scored as "-" when there was no signal above background, "+" when reactivity was weak, and "+ + +" when reactivity was strong.
This is indication of the flagellar nature of the isolated proteins. In addition, the 37, 34 and 32 kD protein and the 44 and 35 kD protein are immunological related.

TABLE 1

| PROTEIN (in kD) | RαTPRTF | RαSAPF | .2A6B4 | .1H11G7 | MCC9 |
|---|---|---|---|---|---|
| 44 | + | + | - | - | - |
| 37 | + + + | + + + | + + + | - | - |
| 35 | - | - | - | - | - |
| 34 | + + + | + + + | + + + | + | - |
| 32 | + + + | + + + | - | + | - |

To further examine the identity and immunological relationship of the proteins present in the flagellar preparation these were blotted on nitrocellulose paper and stained with Coomassie Brilliant blue. Individual bands were excised and used to prepare rabbit antisera. These sera were tested in immunoblots of both *Treponema hyodysenteriae* C5 lysate and purified flagella. Table 2 summarizes the results of these experiments. Reactivity was scored as "-" when there was no signal above background, "+" when reactivity was weak, and "+ + +" when reactivity was strong.

TABLE 2

| PROTEIN (kD) | α 44 | α 37 | α 35 | α 34 | α 32 |
|---|---|---|---|---|---|
| 44 | + + + | - | + + + | – | – |
| 37 | - | + + + | + + + | + + + | + + + |
| 35 | + | - | + + + | - | - |
| 34 | - | + + + | + + + | + + + | + + + |
| 32 | | + + + | + + + | + + + | + + + |

In this table α 44, α 37, α 35, α 34 and α 32 are rabbit immune sera raised against the 44, 37, 35, 34 and 32 kD periplasmic flagellum associated proteins of *Treponema hyodysenteriae* C5 respectively.
The outcome of the immunoblot analysis once more demonstrates that the 44 and 35 kD proteins may share epitopes and that the 37, 34 and 32 kD protein are immunologically related. The reactivity of anti-35 kD serum with the 37, 34 and 32 kD protein is probably due to contamination of the 35 kD preparation with 37 and/or 34 kD protein, since sera raised against the 37, 34 and 32 kD protein do not recognize the 35 kD protein.

Immunogold labeling

To establish if the proteins present in flagellar preparations of *Treponema hyodysenteriae* C5 are exclusively located in the flagella we used the rabbit antisera raised against the individual components of the flagellum in immunogold electron microscopy experiments. Antibodies directed against the 44 kD and 35 kD polypeptide specifically decorated the flagella all over, indicating that these polypeptides are located on the surface of the flagellum. In contrast, antibodies which recognize the 37, 34 and 32 polypeptide react exclusively with the broken ends of flagella and with thin fragments of flagella. This suggests that these antibodies recognize internal flagellar epitopes, exposed only if the external layer is damaged or removed due to breakage of the flagella.

N-terminal amino acid sequencing

All major proteins present in the periplasmic flagella preparation were further characterized by N-terminal amino acid sequencing. The 37, 34 and 32 kD proteins are related since they share a high degree of N-terminal sequence homology. Since their N-terminal sequences are similar but not identical it is likely that these proteins are neither breakdown nor processed products of each other but are encoded by different genes. The N-terminal sequence of the 44 and 35 kD protein were distinct from each other and the other proteins in the flagellar preparation.

Homology

A comparison was made between the N-terminal sequences of the *Treponema hyodysenteriae* flagellar proteins and all available amino acid sequences present in the NBRF (release 28.0) and Swiss prot (release 18.0) databases and the N-terminal amino acid sequences from flagellar proteins of *S. aurantia* - (Parales and Greenberg, 1991). This search revealed no significant homologies with the N-terminal sequences of the 35 and 44 kD proteins. However, the 44 kD protein shared some N-terminal amino acid residues with the sheath proteins of *Treponema pallidum*, *Treponema phagedenis* and *S. aurantia*, which is in accordance with our assumption that the 44 kD protein recides on the surface of the *Treponema hyodysenteriae* flagellum. The 37, 34 and 32 kD protein share a strong similarity with the N-termini of the core polypeptides from other spirochetes, which confirms their flagellar nature.

Antigenic conservation of flagellar proteins within different isolates of *Treponema hyodysenteriae*

We used our monospecific flagella sera to examine the conservation of flagellar antigens from *Treponema hyodysenteriae*. Cell lysates of several isolates and strains of *Treponema hyodysenteriae* were reacted in immunoblots with serum raised against the individual flagellar subunits of *Treponema hyodysenteriae* C5. The treponemes had been characterized previously on blood agar plates as strongly or weakly hemolytic. Only the results of immunoblotting experiments with anti-37 kD and anti-44 kD sera are shown (table 3) since these are representative for results obtained with the anti-37, anti-34, -32 and the anti-44 and -35 kD sera respectively.

TABLE 3

| T. hyodysenteriae isolate/strain | source[a] | hemolysis[b] | antiserum | |
|---|---|---|---|---|
| | | | anti-37[c] | anti-44[d] |
| 27164 | ATCC 27164 | + + + | 37,34,32 | 44 |
| 31212 | ATCC 31212 | + + + | 37,34,32 | 44 |
| 31287 | ATCC 31287 | + + + | 37,34,32 | 44 |
| B8 | FI | + + + | 37,34,32 | 44 |
| C5 | FI | + + + | 37,34,32 | 44 |
| D2 | FI | + /- | 37,34,32 | 47 |
| D7 | FI | + /- | 37,34,32 | 45 |
| PWS/A | Hudson, 1976 | + /- | 37,34,32 | 44 |
| Z7 | FI | + + + | 37,34,32 | 44 |
| Z10 | FI | + + + | 37,34,32 | 44 |
| Z12 | FI | + /- | 37,34,32 | 46 |

[a] FI = Dutch field isolate; ATCC = American Type Culture Collection
[b] + /- = weakly hemolytic; + + + = strongly hemolytic
[c] molecular mass (kDa) of the proteins reactive with rabbit serum to the 37 kDa flagellar protein of *T. hyodysenteriae* C5
[d] molecular mass (kDa) of the proteins reactive with rabbit serum to the 44 kDa flagellar protein of *T. hyodysenteriae* C5. Weak reactivity with a 35 kDa protein was noted in each of the *T. hyodysenteriae* tested.

All isolates tested possessed proteins of 37, 35, 34 and 32 kD which strongly reacted with serum raised against proteins of the same molecular mass in *Treponema hyodysenteriae* C5. Thus these three flagellar proteins appear conserved in *Treponema hyodysenteriae*. Also, in all *Treponema hyodysenteriae* isolates tested a flagellar protein of 35 kD was present. In contrast, the molecular mass of the largest flagellum-associated polypeptide varied between isolates: in strongly hemolytic isolates it was 44 kD, whereas in weakly hemolytic isolates it varied between 40 and 46 kD.

EXAMPLE 2

CLONING AND DNA SEQUENCE ANALYSIS OF A *TREPONEMA HYODYSENTERIAE* GENE ENCODING THE PERIPLASMIC FLAGELLAR SHEATH PROTEIN

Materials and Methods

Bacterial strains and growth coditions

See Example 1. *Escherichia coli* Y1090 was used for λZAP library construction. *E. coli* DH5αF' (Bethesda Research Laboratories Life Technologies, Inc., Gaithersburg, Md.) was used as recipient for plasmid transformations and was grown in LB media or on LB agar plates at 37°C (Maniatis et al., 1982).

Flagella isolation and purification

See Example 1.

Electrophoresis and Western blotting

See Example 1.

Antisera

See Example 1.

N-terminal sequence analysis

See Example 1.

Plasmids and DNAs

Bacteriophage λZAP (Stratagene, San Diego, Calf.) was used for chromosomal-DNA library construction. Plasmid pBluescript II KS$^+$ (Stratagene, San Diego, Calif.) was used for subcloning experiments and in sequencing. Plasmid DNA, chromosomal DNA and phage DNA were isolated according to T. Maniatis, E.F. Fritsch and J. Sambrook (1982) (Molecular Cloning, A Laboratory Manual, Cold Spring Harbor Laboratory, New York). Plasmid DNA for double-stranded sequencing was extracted by using an alkaline lysis procedure and purified by cesium-chloride density gradient centrifugation. *Treponema hyodysenteriae* chromosomal DNA for Southern analysis and library construction was prepared from bacteria harvested from 250 ml cultures and washed twice in phospate buffered saline (PBS).

All restriction endonucleases and DNA-modifying enzymes were used in accordance with the specifications of the manufacturer (Bethesda Research Laboratories, Inc. Gaithersburg, Md., or Boeheringer Mannheim Biochemicals).

Construction and screening of *Treponema hyodysenteriae* chromosomal DNA library.

Chromosomal DNA from *Treponema hyodysenteriae* C5 was partially digested with *Sau*3AI and fragments ranging in size from 6 to 9 kilo base pairs were used to construct a λZAP phage library (R.A. Young and R.W. Davis, 1983 Efficient isolation of genes using antibody probes. Proc. NaH. Acad. Sci. USA. 80, 1194-1198). Briefly, DNA was fractionated by agarose gel electrophoresis, ligated to *Eco* RI adaptors and subsequently packaged as phage according to the protocol of the manufacturer (Stratagene, La Jolla, Calf.). The titer of the library was 1.74x10$^5$ plaque forming units per ml after transfection of competent *E. coli* Y1090 host cells. Approximately 90 % of the phages analyzed were found to contain insertions with an average size of 6 - 9 kilobases. The λZAP library was screened as follows: approximately 90,000 plaques were allowed to grow on *E. coli* Y1090 for 2,5 hour at 42°C, overlayed with nitrocellulose filters (Schleicher and Schuell, Dassel, FRG), previously impregnated with 10 mmol/l IPTG, and further incubated for 4 hours at 37°C. The filters were removed and incubated with antiserum raised against periplasmic flagella from *Treponema hyodysenteriae* diluted 1:500 in PBS$^+$. As secondary antibody alkaline-phosphatase-conjugated goat-anti rabbit antibodies, diluted 1:3000 in PBS$^+$ (Sigma) was used. Immuno reactive plaques were visualized by development in nitro blue tetrazolium (0,37 mmol/l) and 5-bromo-4-chloro-3-indolyl phosphate (0,34 mmol/l) solubilized in 100 mmol/l Tris/HCl, pH 9,5,100 mmol/l NaCl, 5 mmol/l MgCl$_2$.

Affinity purification of antibodies from rabbit-immune serum

Antibodies were affinity purified from rabbit immune serum by binding to the products of plaques fixed to nitrocellulose. Approximately 50,000 plaques were grown on *E. coli* Y1090 on 14 cm LB agarplates at 42°C for 2,5 hours, overlaid with a nitrocellulose filter (Schleicher and Schuell), and incubated at 37°C for 2 hours. The filters were blocked for 1 hour in PBS$^+$ and then incubated for three hours in rabbit immune serum, diluted 1:50 in PBS$^+$. The filter was washed three times 15 min in PBS$^+$. For elution of bound antibody the nitrocellulose filter was incubated with 10 ml 0.2 mol/l glycine, 0.15 mol/l NaCl pH 2.8 for 5 min. The pH of the eluate was immediately readjusted to 8.0 with 5 ml 1 mol/l Tris-HCl pH 8.0. A 1:10 dilution of the eluaat was made in PBS$^+$ and used to probe Western blots.

In vitro transcription/translation of DNA

In vitro transcription/translation of DNA was done with the prokaryotic DNA-directed translation kit from Amersham (Buckinghamshire, England) in the presence of L-$^{35}$S methionine (Amersham, Buckinghamshire, England) according to the protocol of the manufacturer. Molecular weights of the synthesized labeled proteins were estimated by comparison with $^{14}$C methylated protein molecular weight markers (Amersham).

## Southern blot hybridization

DNA fragments were separated in agarose gels and, after depurination, denaturation, and neutralization, transferred to nitrocellulose filters (Hybond-N, Amersham Corp., Arlington Heihts, Ill.) by using a vacuum for 1 hour in 20 X SSPE (1 X SSPE is 0.15 mol/l NaCl, 10 mmol/l sodium phosphate, and 1 mmol/l EDTA, pH 7,4). The DNA was fixed on the filter by exposure to UV for 4 min. Filters were prehybridized in 6 X SSPE, 5 X Denhardt solution (1 x Denhardt solution is 0.02% polyvinyl pyrrolidone, 0.02% BSA and 0.02% Ficoll 400), 0,5% SDS and 100 $\mu$g/ml heat-denatured salmon sperm DNA for 6 hours at 45°C. Radiolabeled probe was added, and hybridization was performed at 45°C for 16 hours. Blots were washed briefly two times in 2 X SSPE, 0,1% SDS at room temperature and twice for 20 minutes at 45°C. Stringency washes of 0.5 X SSPE, 0.1% SDS for 20 minutes were performed. Blots were autoradiographed with intensifying screens at -70°C with Fuji RX film (Fuji Photo Film co., Ltd, Tokyo).

DNA probes for Southern blot analysis were purified by silica gel chromatography (Gene Clean, Bio101, LaJolla, Calf.). Labeling was with $\alpha^{32}$P-dATP (Amersham) by using a random-primed labeling kit (Boehringer Mannheim, FRG).

## DNA sequence analysis

DNA sequencing was done with the T7 sequencing kit from Pharmacia (Uppsala, Sweden) by using the protocol for sequencing double stranded templates. DNA to be sequenced was either a restriction fragment subcloned into pBluescript II KS$^+$ and purified by CsCl density gradient centrifugation or DNA amplified by means of the polymerase chain reaction and purified by silica gel chromatography (Geneclean, Bio101). In addition to the standard M13 primers, synthetic oligonucleotides synthesized on a Gene Assembler Plus (Pharmacia, Uppsala, Sweden) were used in DNA sequence analysis. The sequencing products were separated by denaturing polyacrylamide gel electrophoresis (4,5% polyacrylamide) and detected by autoradiography.

## PCR DNA amplification

Polymerase chain reaction (PCR) DNA amplification was performed by using 2,5 U of *Taq* polymerase (Promega) for 35 cycles with a thermocycler (Bioexcellence, Wessex, Andover Hampshire, England). The PCR was carried out in a volume of 100 ul in the presence of 50 mmol/l KCl, 10 mmol/l Tris/HCl,pH 9.6, 1.5 mmol/l MgCl$_2$, 0.001% gelatine, 0.01% Triton X-100 and 200 umol/l of each dNTP. For amplifying a DNA fragment containing the entire sheath flagellin gene and its regulatory sequences, the T7 primer on λZAP (5' AATACGACTCACTATAG 3') was used in combination with oligonucleotide 5, CCGCTTCCTCCTGAGC 3' (primer 2), derived from partial sequence data obtained from DNA upstream of the flagellin gene. Each PCR cycle consisted of 1 min at 95°C, 1 min at 48°C and 2 min at 72°C.

## Sequence analysis

Nucleotide sequences were analyzed with the PC/Gene (release 6.50; Genofit S.A., Geneva, Switzerland) computing programs.

The FASTA program, release 1.3 (W.R. Pearson and D.J. Lipman. 1988. Improved tools for biological sequence comparison. Proc. Natl. Acad. Sci. USA 85:2444-2448) and the FASTP program, release (D.J. Lipmann and W.R. Pearson. 1985. Rapid and sensitive protein similarity searches. Science 227:1435-1441) were used to compare nucleotide and amino acid sequences with the following data bases: EMBL (release 27.0), GenBank (release 67.0), NBRF/PIR (release 28.0), Swiss-Prot (release 18.0) and Brookhaven (release 4.0). Similar sequences were aligned by using the Clustal computer program (D.G. Higgins and P.M. Sharp. 1988. CLUSTAL: a package for performing multiple alignment on a microcomputer. Gene 73:237-244; D.G. Higgins and P.M. Sharp. 1989. Fast and sensitive multiple sequence alignments on a microcomputer. CABIOS Commun. 5151-153).

## Electron microscopy

Samples of flagellar preparations were applied to pioloform coated copper grids (200 mesh, Biorad) for 10 minutes, then washed briefly 3 times on drops of destilled water and negatively stained with phosphotungsten acid (PTA). Examination was with a Philips 201 electron microscope operating at 60 kV and an objective aperture of 30 $\mu$m.

Results

Cloning of the periplasmic flagellar sheath protein gene

Periplasmic flagella were isolated from *T. hyodysenteriae* strain C5. Electron microscopy was used to confirm that the preparations contained only flagella (not shown). Rabbit sera were raised against the isolated flagella and tested for reactivity on immunoblots to both electrophoretically separated proteins of *T. hyodysenteriae* cell lysates and purified flagella. Sera that specifically reacted with the flagellar proteins were used to screen a *T. hyodysenteriae* library constructed in phage λZAP for flagellar protein expression.

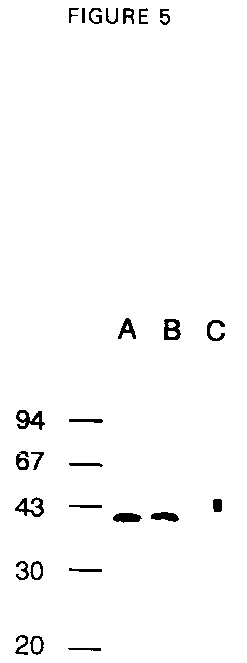

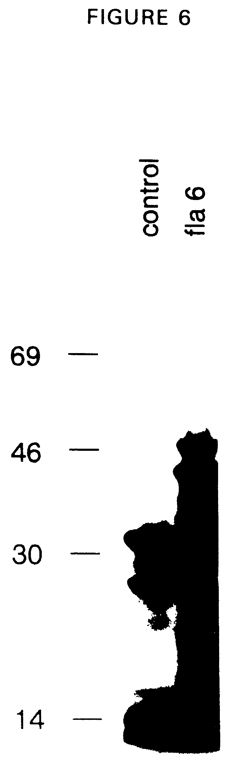

One such screening allowed the identification of six immuno reactive plaques. From one clone the recombinant λZAP phage, designated λZAPII/fla6, was analyzed in further detail. Samples of this phage were deposited with the Centraalbureau voor Schimmelcultures at Baarn (The Netherlands) under No. CBS 513.91. Western blot analysis demonstrated that λZAPII/fla6 directed the synthesis of a 41 kDa polypeptide that strongly reacted with the polyclonal rabbit anti-flagella serum (Fig. 5). Synthesis of immuno-reactive proteins was not induced by isopropyl-$\beta$-thio-galactopyranoside (Fig. 5, lanes A and B), which suggests that *T. hyodysenteriae* expression signals are present on λZAPII/fla6 and functioning in *E. coli*. Antibodies which bound to the proteins expressed by λZAPII/fla6 were affinity purified from rabbit immune serum and used to probe Western blots with purified *T hyodysenteriae* flagella (Fig. 5, lane C). The affinity purified antibodies strongly bound to a 44 kDa protein and weakly to a 35 kDa protein identified as *T. hyodysenteriae* flagellar sheath proteins. The DNA from phage clone λZAPII/fla6 was isolated and digested with the restriction endonuclease *Eco*RI. Agarose gel electrophoresis revealed the presence of two *T. hyodysenteriae* specific *Eco*RI fragments, 1500 and 1750 bp respectively. Both fragments were tested separately in an in vitro transcription translation system to examine whether they directed the synthesis of protein. Only transcription of the 1750 bp fragment resulted in the synthesis of labeled bands, among which a prominent band of approximately 44 kDa (Fig. 6). This molecular mass corresponds with the molecular mass of a *T. hyodysenteriae* sheath flagellin. These results indicate that the 1750 bp fragment contained an entire sheath flagellin gene with its promoter.

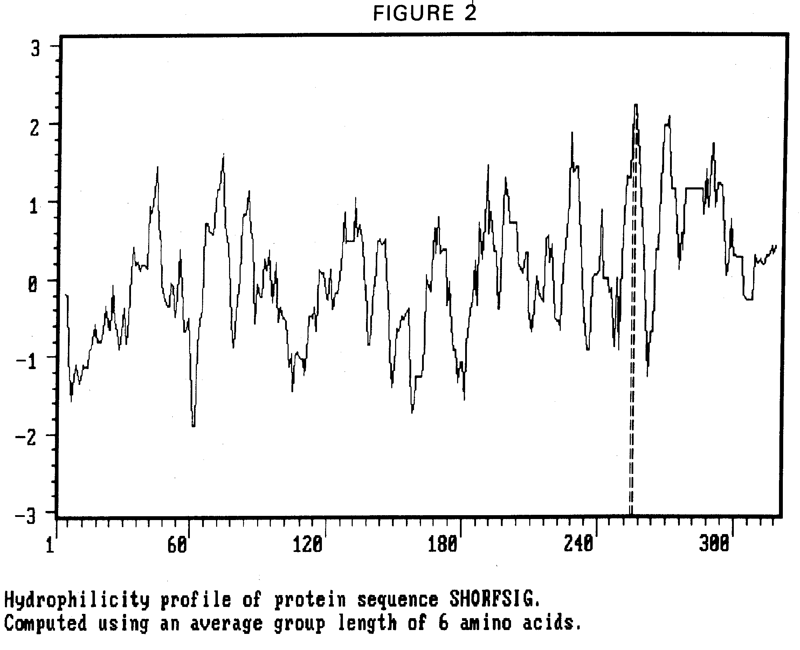

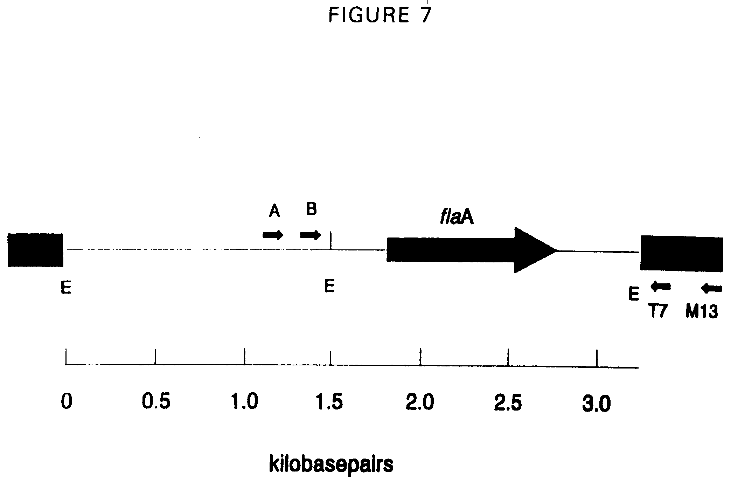

Attempts to subclone the 1750 bp fragment in pBluescript II KS$^+$ were repeatedly negative, suggesting that expression of the gene it contains is toxic in *E. coli*. Therefore, the fragment was amplified by means of the polymerase chain reaction and its nucleotide sequence determined as outlined in Fig. 7. Nucleotide sequence analysis of the amplified DNA revealed the presence of one single open reading frame. The nucleotide sequence encompassing this open reading frame and the derived amino acid sequence are shown in SEQUENCE ID NO: 1. The encoded protein is composed of 320 amino acid residues with a calculated molecular mass of 36.0 kDa. Amino acid residues 20 to 38 correspond to the N-terminal sequence of the purified 44 kDa *T. hyodysenteriae* sheath flagellin. Hence, the open reading frame encodes this *T. hyodysenteriae* sheath flagellin with a 19 amino acid signal peptide. Without the signal peptide, the protein has a calculated molecular weight of 33.8 kDa, which is smaller than the estimate of 44 kDa of the native protein determined by SDS-PAGE. This suggests that the sheath flagellin is post-translationally modified. Hydrophilicity analysis by the algorithm of Kyte and Doolittle (Figure 2) demonstrates one prominent hydrophobic area which is likely to be a membrane spanning domain. This area corresponds with the signal peptide. Upstream from the coding region putative *E. coli*-35 and -10 sequences and a ribosomal binding site are identified. Downstream from the termination codon two inverted repeats occur that may serve as transcription terminator.

Homology analysis of the *T. hyodysenteriae* sheath flagellin

A comparison of both the nucleotide and the deduced amino acid sequence of the sheath protein gene with the sequences present in data bases revealed homology with the sheath flagellins of *T. pallidum* (R.D. Isaacs, J.H. Hanke, L.M. Guzman-Verduzco, G. Newport, N. Agabian, M.V. Norgard, S.A. Lukehart, and J.D. Radolf (1989): Infect. Immun. 57:3403-3411 and R.D. Isaacs and J.D. Radolf (1990): Infect. Immun. 58:2025-2034) and *S. aurantia* (B. Brahamsha and E.P. Greenberg (1989): J. Bacteriol. 171:1692-1697). When both identical amino acids and conserved amino acid substitutions are included the overall sequence similarity of the *T. hyodysenteriae* sheath flagellin to that of *T. pallidum* is 30.5% and to *S. aurantia* is 32.1%.

EXAMPLE 3

**Cloned Flagellar Sheath Protein of *T. hyodysenteriae* Provide Partial Protection against Experimental Disease in Mice.**

**MATERIALS AND METHODS**

**Bacterial strains and growth conditions.**

*T. hyodysenteriae* C5, a Dutch field isolate that is pathogenic for OF-1 mice, was grown as described in EXAMPLE 1. Bacterial concentrations were determined with a hemocytometer under dark-field microscopy. *Escherichia coli* DH5αF'(Bethesda Research Laboratories Life Technologies, Inc., Gaithersburg, Md.) was used as recipient for plasmid transformations and was grown in LB media or on LB agar plates (Maniatis et al., 1982).

**Mice.**

Female Ico: OF1 (OF-1) mice were obtained from Iffa Credo (France) and housed in microisolator cages with free access to standard laboratory food and water.

**Cloning and expression.**

Cloning and expression of the sheath flagellin (flaA) gene of *T. hyodysenteriae* has been described in the foregoing Example. Phage λfla6 containing the flaA gene was used in the polymerase chain reaction (PCR) to obtain a DNA fragment containing the entire reading frame of the gene of interest without adjacent non-coding sequences. Oligodeoxynucleotides (Pharmacia, Woerden, The Netherlands) 5'gggatccccTTAACAAACTCAACTTTG 3' and 5'gggaattcTTATTGAGCTTGTTCTT 3'(nucleotides 160 to 177 and nucleotides 1065 to 1049, respectively in SEQUENCE ID NO: 1 were used to amplify the flagellar sheath gene. Linker sequences, containing a *BamHI* or *EcoRI* restriction enzyme digestion site (designated in lowercase letters in the above oligodeoxynucleotide sequences), were added to facilitate cloning and expression of the amplified gene into plasmid pGEX-3X (Pharmacia) in frame with the glutathion-S-transferase (*Gst*) gene. Ten nanogram of template DNA were used in the PCR (35 cycles) with the following cycle parameters: 1 min at 95°C, 1 min at 48°C, 2 min at 72°C. PCR-amplified DNA segments were isolated by agarose gel electrophoresis, purified by GeneClean (Bio 101, La Jolla, Calif.), digested with EcoRI and BamHI and ligated in pGEX-3X. The recombinant plasmid pGEX-flaA was used to transform *E. coli* DH5α. Expression of fusion protein will then be under control of the *tac* promoter, which is inducible with isopropyl-($\beta$-D-thiogalactopyranoside (IPTG).

The FlaA fusion protein was isolated as follows: 250 ml induced culture of *E. coli* (pGEX/flaA) was centrifuged (6,000 x g, 10 min, 4°C), the bacterial pellet was resuspended in 5 ml phosphate buffered saline (PBS, pH 7.2)/1% Triton, sonicated, and centrifuged (10,000 x g, 20 min, 4°C). The pellet was resuspended in PBS/Triton and sonicated and centrifuged again. This procedure was repeated 4 times. Thus, an insoluble pellet was obtained of which more than 90% of the total protein content consisted of Gst-FlaA, as observed by SDS-polyacrylamide gel electrophoresis (SDS-PAGE) and immunoblotting. This material was used for immunization, Gst-FlaA elctro-eluted from SDS-polyacrylamide gels in a biotrap (Schleicher and Schuell, Dassel, Germany) was soluble and was used in ELISA (see below). Protein concentrations were determined with Protein Assay Reagent (Pierce, Rockford, Ill.) or estimated by comparison with standard amounts of low molecular weight markers (Pharmacia) with SDS-PAGE.

**Vaccination and challenge of OF-1 mice.**

Mice were immunized and challenged by the schedule outlined in Table 4. Herein N is the number of mice per group; ip means intraperitoneal vaccination with Freund's incomplete adjuvant; o + means oral vaccination, in the presence of 10 $\mu$g cholera toxin; o- means oral vaccination, without cholera toxin; For the challenge experiments the doses were administered intragastrically and contained $10^8$ logphase *T. hyodysenteriae* strain C5 (*T.hyo*) or culture medium (TSB)

Table 4

| Immunization and challenge schedule | | | | | |
|---|---|---|---|---|---|
| Group | N | Antigen | Prime (day 0) | Boost (day 21) | Challenge (day 29 and 30) |
| 1 | 12 | - | - | - | T. hyo |
| 2 | 12 | - | - | - | TSB |
| 3 | 12 | Gst | ip | ip | T. hyo |
| 4 | 12 | Gst | ip | o + | T. hyo |
| 5 | 12 | Gst/FlaA | ip | ip | T. hyo |
| 6 | 12 | Gst/FlaA | ip | o + | T. hyo |
| 7 | 7 | water | ip | o + | T. hyo |
| 8 | 5 | Gst/FlaA | ip | o- | T. hyo |

Intraperitoneal (i.p.) immunization occured at the age of 5 weeks with 15 $\mu$g Gst or Gst-Fla in 0.3 ml of Freund's imcomplete adjuvant. Three weeks later mice received either an i.p. boost with the same amount of protein in Freund's incomplete adjuvant or an intragastric dose of 1.0 ml 0.2 mol/l $Na_2HCO_3$ containing 100 $\mu$g Gst or 1 mg Gst-FlaA, with or without 10 $\mu$g cholera toxin (CT; C-3012, Sigma, St. Louis, Mo.). Seven days later mice were intragastrically challenged with $10^8$ *T. hyodysenteriae.* Mice were sacrificed by cervical dislocation 12 days later to evaluate signs of *T. hyodysenteriae* infection in the cecum: catarrhal inflammation, excess intraluminal mucus, oedema, hyperemia and atrophy. Cecal lesions were graded on a scale from 0 to 3:
grade 0 represents lack of lesions;
grade 1 indicates mild lesions;
grade 2 indicates moderate lesions and
grade 3 indicates severe lesions.
Serial dilutions of cecal contents were cultured and colony forming units (cfu) of *T. hyodysenteriae* per gram of cecal material were determined.

**Collection of serum and preparation of gut homogenates.**

From five mice of each group serum was prepared from blood obtained by orbital puncture at day 0 and 21. These mice were sacrificed at day 29 by cervical dislocation and gut homogenates were prepared. The entire intestine was homogenized in an icecold solution of 5 ml 50 mmol/l EDTA containing 0.1 mg/ml soybean trypsin inhibitor (Sigma) and centrifuged at 6000 x g for 10 min at 4 °C. Two ml of supernatant were collected, to which 20 $\mu$l of 100 mmol/l phenylmethylsulphonyl fluoride (PMSF) (Sigma) in 95% ethanol were added and samples were centrifuged at 13,000 x g for 30 min. To 1.5 ml of supernatant, 15 $\mu$l of 100 mmol/l PMSF and 15 $\mu$l of 1 % sodium azide (Sigma) were added and the solution was left at room temperature for 10 min. Subsequently 100 $\mu$l of fetal calf serum were added and the samples were stored at -20 °C.

**Electrophoresis, Western blotting and ELISA.**

Proteins were separated on 12.5% SDS-PAGE, transferred to nitrocellulose sheets, and detected with specific antibodies as described in Example 1. Antibodies against Gst or Gst-FlaA were determined with ELISA. Ninetysix-well polystyrene microtiter plates (Greiner, Alphen a/d Rijn, The Netherlands) were coated overnight at 4 °C with 0.1 ml of a solution containing a previously determined optimal amount of soluble Gst or Gst-FlaA, obtained as described above, in carbonate buffer, pH 9.6. Wells were washed twice with PBS/0.1% Tween 20 (PBST). Fourfold dilutions of 1:20 diluted serum samples or undiluted gut washings were made in PBST and incubated in coated wells for 1 hour at 37 °C. The wells were washed three times with tap water/0.01% Tween 20, and 1:1000 diluted peroxidase-conjugated goat-anti-mouse IgG or peroxidase-conjugated goat-anti-mouse IgA were added. The plates were further incubated for 1 hour at 37 °C. The wells were washed three times with tap water containing 0.01% Tween. The substrate reaction was carried out by adding 100 $\mu$l of a freshly prepared solution containing 50 mg 2,2'-azino-bis (3'ethylbenzthiazolin 6-sulfonic acid and 25 $\mu$l 30% $H_2O_2$ in 100 ml 0.1 mol/l $Na_2HPO_4$-0.05 mol/l citric acid. After 30 min. incubation at room temperature the optical density at 405 nm was measured. Titers were defined as the maximum serum dilution that gave twice the $OD_{405}$ of non-immunized mice.

## RESULTS

### Production and purification of recombinant proteins.

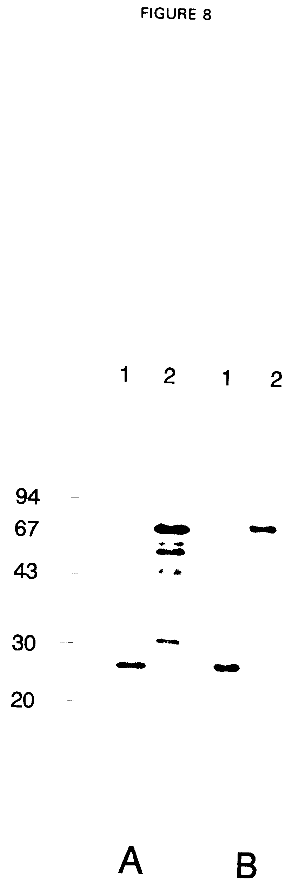

Recombinant Gst and Gst-FlaA were purified from *E. coli* and analyzed by SDS-PAGE and immunoblotting to determine the purity of the preparations (fig. 8, panels A and B, respectively). No contaminating proteins were detected in preparations of Gst: on immunoblots all protein bands in these preparations reacted with specific antisera against Gst (Panel B, lane 1). Preparations of Gst-FlaA contained a major 68 kDa band and fainter bands of smaller molecular mass on Coomassie Brilliant Blue stained SDS-polyacrylamide gels. The majority of the smaller proteins are breakdown products of the Gst-FlaA fusion product, since they react with antisera against Gst (Panel B, lane 2).

### Antibody response to *T. hyodysenteriae* recombinant proteins.

The murine immune response to i.p. administration of Gst or Gst-FlaA in Freund's incomplete adjuvant, followed by either an i.p. boost of antigen in Freund's incomplete adjuvant or an oral boost in the presence of cholera toxin was investigated. In Table 5 the groups are the same as defined in Table 4; the results are expressed as mean antibody titer per group of five mice. ND means not determined.

Table 5

| Group | serum IgG αGst | gut IgA αGst | serum IgG αGst-FlaA | gutIgA αa GSt-FlaA |
|-------|----------------|--------------|---------------------|--------------------|
| 1 | 0 | 0 | 0 | 0 |
| 2 | 0 | 0 | 0 | 0 |
| 3 | 140,000 | 23 | ND | ND |
| 4 | 57,000 | 1 | ND | ND |
| 5 | ND | ND | 62,000 | 1 |
| 6 | ND | ND | 180 | 1 |
| 7 | 0 | 0 | 0 | 0 |
| 8 | ND | ND | 170 | 1 |

It can be concluded from Table 5 that i.p. immunization with Gst-FlaA followed by an i.p. boost induced both detectable serum IgG antibodies and gut-IgA antibodies specific for Gst-FlaA. When the i.p. prime was followed by an oral boost in the presence of cholera toxin, the serum IgG antibody response was significantly less, whereas specific IgA could not be detected. An oral boost of Gst-FlaA without CT had a similar effect on the IgA and IgG titers. In non-immunized mice specific antibodies were not detected, neither in the serum, nor in the intestine.

### Challenge with *T. hydodysenteriae*.

To determine the role of flagellin in protection, immunized mice and control mice were challenged orally with *T. hyodysenteriae* and evaluated for the presence of lesions in the cecum (Table 6). The group numbers in Table 6 correspond with those in Table 4. Each group consisted of seven mice. The results are expressed as group mean cecal score, plus or minus the standard deviation.

Table 6

| Group | cecal score |
|---|---|
| 1 | 2.4 ± 0.7 |
| 2 | 0 ± 0 |
| 3 | 2.0 ± 0.3 |
| 4 | 2.1 ± 0.6 |
| 5 | 1.6 ± 0.7 |
| 6 | 1.9 ± 1.0 |
| 7 | 2.2 ± 0.9 |

Non-immunized mice (group 1) or mice immunized with Gst (group 3 and 4) or water (group 7) had moderate to severe cecal lesions. Ceca were less affected in mice immunized twice i.p. with Gst-FlaA (group 5). Mice immunized i.p with Gst-FlaA and boosted orally with antigen in the presence of cholera toxin had cecal comparable to those observed in the control groups (group 6). Mice primed i.p. with Gst-FlaA and orally boosted with Gst-FlaA without cholera toxin were not protected against subsequent challenge with *T. hyodysenteriae* (group 8). Non-immunized mice inoculated with culture medium had no cecal lesions (group 2).

## EXAMPLE 4

**Immunization of Mice by Attenuated Salmonella typhimurium Expressing *T. hyodysenteriae* Antigens.**

**MATERIALS AND METHODS**

**Bacterial strains.**

The vehicles used for the delivery of *T. hyodysenteriae* antigens to mice were *Salmonella typhimurium* SL3261, an aroA mutant kindly provided by B.A.D. Stocker, and S. typhimurium X3987, a Δcya Δcrp Δasd mutant. X3987 has a deletion of the chromosomal gene for $\beta$-aspartate semialdehyde dehydrogenase (Δasd) so that it is unable to grow in the absence of diaminopimelic acid (DAP), an essential constituent of the cell wall. *E. coli* DH5αF' (Bethesda Research Laboratories Life Technologies, Inc., Gaithersburg, Md.) was used as a transformation recipient of plasmid DNA. *E. coli* Δasd X6097 was used as an intermediate recipient of plasmid pYA292 and derivatives thereof.

**Culture conditions**.

*S. typhimurium* and *E. coli* were grown in LB broth or on LB plates at 37°C containing 50 $\mu$g kanamycin/ml or 100 $\mu$g ampicillin/ml when appropriate [J. Sambrook, E.F. Fritsch, and T. Maniatis. 1989. Molecular cloning: a laboratory manual. Cold Spring Harbor Laboratory. Cold Spring Harbor, N.Y.]. Inocula for oral immunization with *S. typhimurium* were prepared by culturing strains in 500 ml LB (with the appropriate antibiotic) overnight at 37°C with shaking (200 rpm). Cells were harvested by centrifugation (10,000 x g, 10 min, 4°C) and resuspended in 2 ml PBS, pH 7.2 to give a concentration of approximately $10^{10}$ colony forming units (cfu) per ml.

**Plasmids**.

Plasmid pGEX-3X (Pharmacia) carries the gene for glutathion-S-transferase (Gst) under the control of the isopropyl-$\beta$-D-thiogalacto pyranoside (IPTG)-inducible tac promoter. Plasmid pUEX2 (Amersham, Buckinghamshire, UK) encodes-$\beta$-galactosidase and is temperature controlled by the cro-cl857 gene. Plasmid pYA292 is an asd + cloning vector in which cloned antigens are under control of the Ptrc promoter [Nakayama, K., S.M. Kelly, and R. Curtiss III. 1988. Construction of an Asd[+] expression-cloning vector: stable maintenance and high level expression of cloned genes in a *Salmonella* vaccine strain. Bio/Tech.

15

6:693-697]. The vector complements the chromosomal Δasd deletion of X3987 and thus is stably maintained in X3987 when grown in the absence of DAP, e.g. in mammals, were DAP is not present. Plasmid pLUC108.2 contains the multiple cloning site and the first bases of LacZα gene from pUC8.2 [Z. Hanna, C. Fregau, G. Préfontaine, and R. Brousseau. 1984. Construction of a family of universal expression plasmid vectors. Gene 30:247-250.], cloned into plasmid pLG338, which is a derivative of plasmid pSC105 [N.G. Stoker, N.G., N.F. Fairweather, and B.G. Spratt. 1982. Versatile low-copy-number plasmid vectors for cloning in *Escherichia coli* Gene 18:335-341.]. The plasmid is present at 6 to 8 copies per cell. Expression is from the lac promoter.

**Construction of vectors containing a *T. hyodysenteriae* sheath flagellar gene.**

The flagellar sheath protein gene *flaA* of *T. hyodysenteriae* were cloned and their nucleotide sequence determined as described. Chromosomal DNA was isolated as described in Examples 1 and 2. Ten nanograms of phage λfla6 containing the *flaA* gene was used in the polymerase chain reaction (PCR) to obtained a DNA fragment containing the entire reading frame of the gene of interest. Oligonucleotides (Pharmacia, Woerden, The Netherlands) used for amplification were based on the known nucleotide sequence of *flaA* and were flanked by a linker sequence containing a *Bam*HI or *Eco*RI restriction enzyme degestion site to facilitate cloning and expression. Primers 5'TTAACAAACTCAACTTTG 3' (nucleotide 160-177 in SEQUENCE ID NO.: 1) and 5'TTATTGAGCTTGTTCTT3' (nucleotide 1049-1065) were used to amplify a fragment containing the *flaA* gene sequence. Cycle parameters were: 1 min at 95°C, 1 min at 35°C and 2 min at 72°C. PCR-amplified DNA fragments were purified by GeneClean, digested with EcoRI and BamHI and ligated into plasmid pYA292, pLUC108.2, pGEX-3X and pUEX2. In pYA292 a non-fusion protein is encoded. With pLUC108.2 a fusion to the lacZα peptide (1.2 kD) is made, whereas with pGEX-3X a fusion protein to Gst (27.5 kD) is made and with pUEX2 a fusion with β-galactosidase (116 kD).

**E. coli and S. typhimurium genetic manipulations**.

Recombinant plasmids containing *flaA* were used to transform *E. coli* by electroporation [W.J. Dower, J.F. Miller, and W. Ragsdale. 1988. High efficiency transformation of *E. coli* by high voltage electroporation. Nucleic Acids Res. 13:6127-6144.] Plasmids encloding production of recombinant sheath flagellin (*FlaA*) were purified from *E. coli* by cesium chloride gradient centrifugation and used to transform *S. typhimurium* by electroporation. Transformants were screened for antibiotic resistance (pLUC108.2, pGEX-3X, pUEX2) or growth in the absence of DAP (pYA292). Transformants were tested for recombinant *FlaA* production by immunoblot analysis of 50 μl of a 40 ml overnight culture washed once in phosphate buffered saline, pH 7.0 (PBS). To verify that electroporation of *S. typhimurium* had not selected for rough mutants, the LPS profile of transformants was examined by using SDS-PAGE and silver staining [P.J. Hitchcock, and T.M. Brown. 1983. Morphological heterogeneity among *Salmonella* lipopolysaccharide chemotypes in silver-stained polyacrylamide gels. J. Bacteriol. 154:269-277.]

**In vitro stability of plasmids**.

Overnight cultures of *S. typhimurium* transformant in LB broth were diluted 100-fold in fresh medium and incubated for an additional 8 hour. Subsequently, these cultures were diluted again 100-fold in fresh medium and incubated overnight. Serial dilutions of each subculture were plated on LB plates with and without the appropriate antibiotic.

**Immunization of mice, enumeration of bacteria in mouse organs, collection of sera and gut washings**.

BALB/c females of 10 weeks of age were orally dosed using a gavage needle with $10^{10}$ cfu of the *S. typhimurium* vaccine strain at day 0 and day 21. At 7, 14 and 21 days after the first dose, groups of 3 animals were sacrificed and the number of Salmonellae present in the livers and spleens was determined. Livers and spleens were homogenized in sterile distilled water using a stomacher. Viable counts were performed on these homogenates in duplicate on LB plates supplemented with and without antibiotic to determine the retention of the recombinant plasmids in the colonies recovered from mice immunized with strains containing pLUC108.2, pGEX-3X or pUEX2 plasmid. Expression of *FlaA* in these recombinant *S. typhimurium* was examined. Nitrocellulose filters (Hybond C. Amersham) were laid over LB plates and recovered cells were streaked onto the nitrocellulose. After overnight incubation at 37°C the filters were lifted, exposed to chloroform vapour for 1 hour and washed 4 times in PBS containing 0.05% Tween. Immunoscreening of the filterlifts was performed with specific antisera against *FlaA* similar as with immunoblots (see below). In mice sacrificed at day 42 serum and gut antibody response to *S. typhimurium* and *T. hyodysenteriae* antigens was determined with ELISA. Blood samples were collected by tail bleeds, or cardiac puncture from mice anaesthesized with halothane, 3 weeks after the first and second oral doses. Blood was mixed with an equal volume of a solution of 85 mmol/l tri-sodium citrate-65 mmol/l citric acid, centrifuged at 6000 x g and supernatant was stored at -20°C. Gut samples were prepared from minced small intestine as described in Example 3.

## SDS-PAGE, immunoblotting (Western blotting) and ELISA.

Proteins were separated on 12.5% SDS-polyacylamide gels (SDS-PAGE) and visualized by Coomassie Brilliant Blue R 250 staining. After electrophoretic transfer to nitrocellulose filters, proteins were reacted with specific antiserum and bound antibodies were visualized with alkaline phosphatase conjugated antibodies as described. Antibodies specific for recombinant *FlaA* were detected in ELISA as described in Example 3. Titers were measured as the $OD_{405}$ of 1/750 dilutions of sera or 1/16 dilutions of gut homogenates, minus the avarage values obtained with four non-immunized mice.

## Immunological reagents.

Polyclonal antiserum against *FlaA* was prepared by immunizing rabbits with denatured *FlaA* isolated from Western blots Gst was obtained by affinity chromatography from *E. coli* expressing these antigens according to Example 3. One hundred μg purified Gst was emulsified in Freund's complete adjuvant in a final volume of 3 ml and injected subcutaneously and intramuscularly into New Zealand White rabbits on day 0 and day 21. Serum collected at day 34 was used in immunoblotting experiments and in ELISA.

## RESULTS

### In vitro expression of recombinant *T. hyodysenteriae* proteins by attenuated *S. typhimurium*.

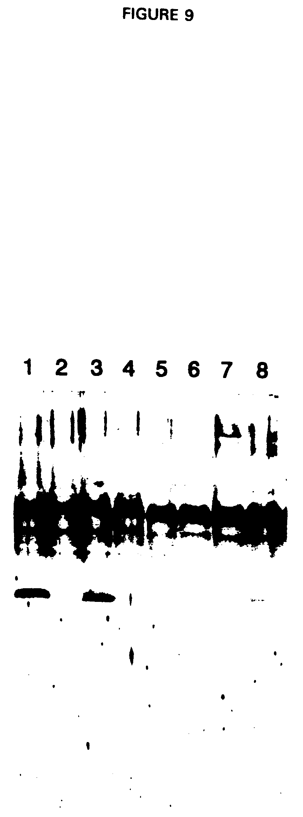

Plasmids directing the synthesis of *T. hyodysenteriae FlaA* were transformed to *S. typhimurium* SL3261 (plasmids pLUC108.2, pGEX-3X, pUEX2) or *S. typhimurium* X3987 (plasmid pYA292) by electroporation. All transformants tested were smooth as judged by LPS specific staining of proteinase K treated whole cell lysates (not shown) and expressed recombinant *FlaA* as shown by immunoblotting (Fig. 9). Because SL3261 and X3987 do not carry the *lacI* gene on the chromosome protein expression with pGEX-3X, pLUC108.2 and pYA292 plasmids is constitutive. Heterologous antigen expression by bacteria containing pUEX2 plasmid was constitutive as well, since these bacteria were grown at the permissive temperature. Molecular mass of recombinant *FlaA* (fusion) protein encoded by pLUC108.2. pYA292, PGEX-3X and pUEX2 is 40, 39, 66 and 156 kDa respectively (Fig. 9). Important differences in level of expression were observed. With pGEX-3X *FlaA* synthesis could barely be detected on immunoblots. Noteworthy, these *S. typhimurium* grew slower than the other salmonella. Apparently the expression of *FlaA* is deleterious to the host. With none of the vaccine strains expression of recombinant protein was detected on Coomassie Brilliant Blue stained gels.

Plasmid pYA292 is stably maintained in *T. hyodysenteriae* X3987. In order to determine the in vitro stability of the other plasmids, vaccine strains were passaged repeatedly in medium without antibiotic. When serial dilutions of the subcultures were plated on LB plates with and without appropriate antibiotic, the presence of antibiotic had no significant effect on the number of cfu obtained with *S. typhimurium* SL3261 containing pGEX-3X, pLUC108.2 or derivatives, thus demonstrating reasonable stability of these plasmids in the host bacteria under these conditions.

### Colonization of liver and spleen of mice orally infected with Salmonella vaccine strains.

BALB/c mice were dosed orally with $10^{10}$ cfu of *S. typhimurium* vaccine strain producing recombinant *FlaA* protein. At day 7, 14 and 21 after infection livers and spleens were examined for the presence of vaccine strain (Table 7).

Table 7

| vaccine | mouse | no. of cfu | | | no. of antibiotic resistant cfu | | |
|---|---|---|---|---|---|---|---|
| | | day 7 | day 14 | day 21 | day 7 | day 14 | day 21 |
| pYA292 | 1 | 4500 | ND | ND | ND | ND | ND |
| | 2 | 6300 | ND | ND | ND | ND | ND |
| | 3 | 3700 | ND | ND | ND | ND | ND |
| Pya292/flaA | 1 | 0 | 0 | 0 | ND | ND | ND |
| | 2 | 0 | 0 | 0 | ND | ND | ND |
| | 3 | 0 | 0 | 0 | ND | ND | ND |
| pLUC | 1 | 5800 | ND | ND | 4900 | ND | ND |
| | 2 | 1800 | ND | ND | 1400 | ND | ND |
| | 3 | 0 | ND | ND | 20 | ND | ND |
| pLUC/flaA | 1 | 0 | 0 | 0 | 0 | 0 | 0 |
| | 2 | 0 | 0 | 0 | 0 | 0 | 0 |
| | 3 | 0 | 0 | 0 | 0 | 0 | 0 |
| pGEX | 1 | 600 | ND | ND | 700 | ND | ND |
| | 2 | 40 | ND | ND | 10 | ND | ND |
| | 3 | 10 | ND | ND | 10 | ND | ND |
| pGEX/flaA | 1 | 150 | 25 | 0 | 0 | 15 | 0 |
| | 2 | 0 | 0 | 0 | 0 | 0 | 0 |
| | 3 | 0 | 0 | 0 | 0 | 0 | 0 |

The results in Table 7 are expressed as colony forming units (cfu) per organ, and were determined in the presence or absence of the appropriate antibiotic. ND means not determined.

Vaccine strains containing pGEX, pYA292 or pLUC without insert were only monitored at day 7. Results obtained with livers are shown in Table 7 and are similar as those obtained with spleens. At day 7 X3987 (pYA292) was found in livers in high numbers (respectively $3.7 \times 10^3$ or more, and $4.5 \times 10^3$ or more bacteria per organ). Similar numbers of SL3261 containing pLUC were detected in livers of two mice; in the third mouse no salmonellae were detected. With *S. typhimurium* containing pGEX 10 to 600 bacteria per liver were isolated. No salmonellae carrying pLUC/*flaA* or pGEX/*flaA* were ever reisolated from livers or spleens. All salmonellae isolated from livers and spleens expressed the cloned antigen, except for salmonellae that were isolated from a mouse immunized with SL3261 (pGEX/*flaA*). All salmonellae isolated at day 7 from this animal had lost the plasmid, whereas at day 14 almost half of the recovered salmonellae did not contain the plasmid.

**Murine antibody response to *S. typhimurium* and *T. hyodysenteriae* antigens.**

Serum and gut homogenates from mice killed 3 weeks after they had received an oral boost of $10^{10}$ salmonellae vaccine strain were analyzed by ELISA for antibodies against whole salmonellae sonicate, recombinant *FlaA*. All mice had developed an immunoglobulin anti-salmonella titer in gut homogenates, though the degree of response varied between vaccine constructs (Table 8).

## Table 8

| gut IgA antibody | | | | | | |
|---|---|---|---|---|---|---|
| mouse | pYA292 | pYA292/flaA | pLUC | pLUC/flaA | pGEX | pGEX/flaA |
| 1) | 1.407 | 0.656 | 1.439 | 1.164 | 1.180 | 0.384 |
| 2) | 0.808 | 0.510 | 1.570 | 0.716 | 0.298 | 0.289 |
| 3) | > 2 | 0.739 | 1.299 | 1.365 | 0.146 | 1.468 |
| 4) | > 2 | 0.785 | 0.831 | 1.468 | 0.900 | 0.343 |
| serum IgG antibody | | | | | | |
| mouse | pYA292 | pYA292/flaA | pLUC | pLUC/flaA | pGEX | pGEX/flaA |
| 1) | 0.725 | 0.537 | 0.564 | 0 | 0.235 | 0.047 |
| 2) | 0.759 | 0.592 | > 2 | 0 | 0.246 | 0.145 |
| 3) | > 2 | 0.869 | > 2 | 0 | 0.304 | 0.203 |
| 4) | > 2 | 0.441 | 0.371 | 0.232 | 0.147 | 0.145 |

The results in table 8 are given as $OD_{405}$ of 1:16 diluted gut washings or 1:750 diluted serum samples for each of four mice of each group of animals, minus the average value obtained with four non-immunized animals (0.207, and 0,284 respectively).

All three pLUC constructs induced potent gut antibody responses to salmonella sonicates, whereas the response induced by X3987 (pYA292/*flaA*) was markedly reduced compared with X3987 (pYA292). The responses to the pGEX vaccines were different again, 3 of the 4 mice responded poorly to the S13261 (pGEX) and SL3261 (pGEX/*flaA*) vaccines.

As seen in the gut, the serum antibody responses to salmonellae sonicates varied between vaccine constructs, and were not directly related to the relative magnitude of the responses seen in the gut (Table 8). Highest serum responses were found in mice immunized with X39879(pYA292) or SL3261 (pLUC) whereas oral immunization of mice with SL3261 containing pGEX derivatives or pLUC/*flaA* resulted in a serum anti-salmonella response just above those measured in non-immunized animals.

SEQUENCE LISTING

INFORMATION FOR SEQUENCE ID NO: 1

SEQUENCE CHARACTERISTICS:

    (A) LENGTH: 1140 base pairs

    (B) TYPE: nucleic acid

    (C) STRANDEDNESS: double

    (D) TOPOLOGY: linear

MOLECULE TYPE: cDNA to genomic RNA

ORIGINAL SOURCE:

    (A) ORGANISM: Treponema hyodysenteriae

    (B) STRAIN: C 5

IMMEDIATE EXPERIMENTAL SOURCE

NAME OF CELL LINE: CBS 513.91

FEATURE 1:

    (A) LOCATION: from 1 to 102 base pairs

PROPERTIES: NON CODING REGION

FEATURE 2:

    (A) LOCATION: from 103 to 1062 pairs

    (B) OTHER INFORMATION: open reading frame

PROPERTIES: endoflagelar sheath protein

FEATURE 3:

    (A) LOCATION: from 103 to 159 base pairs

    (B) OTHER INFORMATION: open reading frame

PROPERTIES: signal peptide

FEATURE 4:

    (A) LOCATION: from 159 to 1062 base pairs

    (B) OTHER INFORMATION: open reading frame

PROPERTIES: mature endoflagelar sheath protein

FEATURE 5:

    (A) LOCATION: from 27 to 32 base pairs

PROPERTIES: putative -35 region

FEATURE 6:

    (A) LOCATION: from 50 to 55 base pairs

PROPERTIES: putative -10 region

FEATURE 7:

    (A) LOCATION: from 86 to 91 base pairs

    PROPERTIES: putative ribosome binding site


SEQUENCE DESCRIPTION: SEQUENCE ID NO: 1


```
TGATTTATTT TTTTGATTAA ATTTGCTTGA CAAATTAAGA AATTTAGTGT ATAAAAGTTG          60

TATTATTATG CTTATTAATT AGTACAAGGA GTGCAATAAA TT ATG AAA AAG TTA TTC GTA    120
                                             Met Lys Lys Leu Phe Val
                                              1               5

GTA TTA ACT TCC ATT TTT ATC GCT GCA TCT GCT TAC GGT TTA ACA AAC          168
Val Leu Thr Ser Ile Phe Ile Ala Ala Ser Ala Tyr Gly Leu Thr Asn
            10              15                  20

TCA ACT TTG ATT GAT TTT GCT TTA ACA GGT AAT GCT GAT AAC TTA CAA          216
Ser Thr Leu Ile Asp Phe Ala Leu Thr Gly Asn Ala Asp Asn Leu Gln
        25              30                  35

GCT GGA GAA GGT GAT ACA AAT GAA GTA GTT CCA GTT GCA GAA AAT CTT          264
Ala Gly Glu Gly Asp Thr Asn Glu Val Val Pro Val Ala Glu Asn Leu
    40              45                  50

TAT AAT GAT AAC TGG GTA GTA TGG TTG AAT GAA TCT GCT AGA TTA ACA          312
Tyr Asn Asp Asn Trp Val Val Trp Leu Asn Glu Ser Ala Arg Leu Thr
55                  60              65                  70

GAG AAT CGC AGA AAT TCT TAT GTT ACT AAC GTA GAC AGT AAA GGT AAC          360
Glu Asn Arg Arg Asn Ser Tyr Val Thr Asn Val Asp Ser Lys Gly Asn
            75              80                  85

AAC GGT GCT TGG GAA GCA GGT AAA GTT CTT GGT GTA AGA GTA CAT TTC          408
Asn Gly Ala Trp Glu Ala Gly Lys Val Leu Gly Val Arg Val His Phe
            90              95                  100

CCA TTA GCA GCT TGG AAC AGT TAT GCT TTA GTA AAA CCA GTA TAT GAA          456
Pro Leu Ala Ala Trp Asn Ser Tyr Ala Leu Val Lys Pro Val Tyr Glu
        105             110                 115

CTT GAA ATG TAT GGC GGT GCT GAT GGT ACT AAA TAT ACA GAA GGT AAA          504
Leu Glu Met Tyr Gly Gly Ala Asp Gly Thr Lys Tyr Thr Glu Gly Lys
        120             125                 130

GGT GTT ATA CAT AAC GTT GGC GAA ATC AAA TCT ATT AGT TCT TGG GTT          552
Gly Val Ile His Asn Val Gly Glu Ile Lys Ser Ile Ser Ser Trp Val
135                 140                 145                 150

TAT GGA CGT AAC TAT TTA ATT AGT TAT TTC GTA AAC TTA CAA AAT GAA          600
Tyr Gly Arg Asn Tyr Leu Ile Ser Tyr Phe Val Asn Leu Gln Asn Glu
            155             160                 165
```

```
TTT GGT GAA TTA AAA TCT TAT CCA ATG GGT ACT GTT TAC TTC AAC GGT      648
Phe Gly Glu Leu Lys Ser Tyr Pro Met Gly Thr Val Tyr Phe Asn Gly
            170                 175                 180

TGG AGA CAA GTA AGA TGG GAA AAC AGA GAA TAC TTA CCT AAT GTT CGC      696
Trp Arg Gln Val Arg Trp Glu Asn Arg Glu Tyr Leu Pro Asn Val Arg
            185                 190                 195

GAC AGA GTA TTA GTA AGA GAA CCT CTT TAT CCT AGA ATG ATC CCT TCT      744
Asp Arg Val Leu Val Arg Glu Pro Leu Tyr Pro Arg Met Ile Pro Ser
            200                 205                 210

GTT AAA TTA GAT TCT TTA GGT TTC TAT AGA ACT AAA GAT ACT AAA GGC      792
Val Lys Leu Asp Ser Leu Gly Phe Tyr Arg Thr Lys Asp Thr Lys Gly
215                 220                 225                 230

GGA GAT TTC ATC ACT TAC GTT AAA GAT GTA ACA CTT GAG TAT GAC GTA      840
Gly Asp Phe Ile Thr Tyr Val Lys Asp Val Thr Leu Glu Tyr Asp Val
                235                 240                 245

GTA GTT GTT GAT TTT GAA GAA GAT ATC GAC GAT GAA GCT ACT TGG CAG      888
Val Val Val Asp Phe Glu Glu Asp Ile Asp Asp Glu Ala Thr Trp Gln
            250                 255                 260

TTA TTA AAA ACA GAA AAC GAT AGA AAA CAA GCT ATC GAA TCT GCT AGA      936
Leu Leu Lys Thr Glu Asn Asp Arg Lys Gln Ala Ile Glu Ser Ala Arg
            265                 270                 275

ATA CGT GAA CAA GCT GAG TTA AGA GAT CTT GAA CAA AGA CGT ATA GGC      984
Ile Arg Glu Gln Ala Glu Leu Arg Asp Leu Glu Gln Arg Arg Ile Gly
            280                 285                 290

GAC GGT ACT GCT GCT GAT CAA GGT GCT GCT GCT AAT ACA GGT GCT GCT     1032
Asp Gly Thr Ala Ala Asp Gln Gly Ala Ala Ala Asn Thr Gly Ala Ala
295                 300                 305                 310

GAC ACA GGC GCT GCT CAA GAA CAA GCT CAA TAATTAAATT AGTTTGGAAA      1082
Asp Thr Gly Ala Ala Gln Glu Gln Ala Gln     -
            315                 320

TAAAAGGCTA ATTAATAGAT TAAGGGGAAA CTTTAATAGT TTCCCCTTTT TTTATTGT     1140
```

## Claims

1. Polynucleotide comprising a nucleotide sequence coding for at least part of the endoflagellum sheath protein of Treponema hyodysenteriae.

2. Polynucleotide selected from:
   a. DNA having at least part of the nucleotide sequence represented in SEQUENCE ID NO: 1;
   b. polynucleotides which hybridize to any of the foregoing DNA and which codes for a polypeptide having immunogenic properties of the endoflagellum sheath protein of Treponema hyodysenteriae;
   c. polynucleotides which are degenerate as a result of the genetic code to the DNA as defined in (a) or the polynucleotides as defined in (b) and which code for a polypeptide having immunogenic properties of the endoflagellum sheath protein of Treponema hyodysenteriae.

3. Recombinant polynucleotide coding for the expressing of at least part of the endoflagellum sheath protein of Treponema hyodysenteriae, characterized in that it contains a suitable vector and a polynucleotide according to claim 1 or 2 inserted therein.

4. Polypeptide having at least part of the amino acid sequence of the endoflagellum sheath protein of Treponema hyodysenteriae.

5. Polypeptide having at least part of the amino acid sequence represented in SEQUENCE ID NO: 1.

6. Vaccine for combating Treponema hyodysenteriae infection containing an effective amount of a recombinant polynucleotide according to claim 3 or a polypeptide according to claim 4 or 5.

7. Use of a polypeptide according to claim 4 or 5 in combating Treponema hyodysenteriae infections.

FIGURE 1

Hydropathic index of SHORFSIG from amino acid 1 to amino acid 320.
Computed using an interval of 9 amino acids. (GRAVY = -4.35).

EP 0 534 526 A1

FIGURE 2

Hydrophilicity profile of protein sequence SHORFSIG.
Computed using an average group length of 6 amino acids.

EP 0 534 526 A1

# FIGURE 3

Plot of the probability profiles in the 3 conformations for sequence SHORFSIG.
Extent of regions predicted in a conformation are indicated on top of graphs.

FIGURE 4

Plot of secondary structure curves for sequence SHORFSIG.
From position 1 to 320.

EP 0 534 526 A1

FIGURE 5

A  B  C

94 —

67 —

43 —

30 —

20 —

FIGURE 6

## FIGURE 7

kilobasepairs

FIGURE 8

FIGURE 9

| | European Patent Office | PARTIAL EUROPEAN SEARCH REPORT which under Rule 45 of the European Patent Convention shall he considered, for the purposes of suhsequent proceedings, as the European search report | Application Number EP 92 20 2796 |

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 5) |
|---|---|---|---|
| X | JOURNAL OF GENERAL MICROBIOLOGY vol. 135, no. 6, 1989, pages 1625 - 1632 K. KENT ET AL 'Analysis of the axial filaments of Treponema hyodysenteriae by SDS-PAGE and immunoblotting' | 4-7 | C12N15/31 A61K39/02 C07K13/00 |
| Y | * the whole document * | 1-3 | |
| Y | WO-A-9 015 132 (SYNTRO CORPORATION) 13 December 1990 | 1-3 | |
| A | * the whole document * | 4-7 | |
| P,X | INFECTION AND IMMUNITY. vol. 60, no. 7, July 1992, WASHINGTON US pages 2920 - 2925 M. KOOPMAN ET AL 'Cloning and DNA sequence analysis of a Serpulina (Treponema) hyodysenteriae gene encoding a periplasmic flagellar sheath protein' * the whole document * | 1-7 | |
| | ----- | | TECHNICAL FIELDS SEARCHED (Int. Cl. 5) C07K |

## INCOMPLETE SEARCH

The Search Division considers that the present European patent application does not comply with the provisions of the European Patent Convention to such an extent that it is not possihle to carry out a meaningful search into the state of the art on the basis of some of the claims
Claims searched completely :
Claims searched incompletely :
Claims not searched :
Reason for the limitation of the search:

see sheet C

| Place of search | Date of completion of the search | Examiner |
|---|---|---|
| THE HAGUE | 05 JANUARY 1993 | VAN DER SCHAAL C.A. |

EPO FORM 1503 03.82 (P04E07)

Sheet C                                        EP 92202796


Remark:
Although claim 7 is directed to methods of treatment of the animal body
the search has been carried out and based on the alleged effects of the
compound.